Europäisches Patentamt

European Patent Office

Office européen des brevets

(19)

⌢ Publication number: **0 298 685**

**A1**

## (12) EUROPEAN PATENT APPLICATION

(21) Application number: **88306080.8**

(22) Date of filing: **04.07.88**

(51) Int. Cl.⁴: **A61F 5/42**

(30) Priority: **09.07.87 GB 8716170**

(43) Date of publication of application:
**11.01.89 Bulletin 89/02**

(84) Designated Contracting States:
**AT BE CH DE ES FR GB IT LI LU NL SE**

(71) Applicant: **CRAIG MEDICAL PRODUCTS LIMITED**
**204a London Road**
**East Grinstead Sussex RH19 1EY(GB)**

(72) Inventor: **Steer, Peter Leslie**
**Kingscote Woodlands Rise**
**East Grinstead Sussex(GB)**

(74) Representative: **Cook, Anthony John et al**
**D. YOUNG & CO. 10, Staple Inn**
**London, WC1V 7RD(GB)**

(54) Condom applicator.

(57) A male prophylactic condom is vacuum drawn into close contact with the internal walls of an applicator. At the time of use, the partial vacuum between the condom and the applicator is destroyed causing the condom to grip the penis.

EP 0 298 685 A1

## CONDOM APPLICATOR

With the present worrying spread of sexually transmitted diseases such as AIDS (Acquired Immune Deficiency Syndrome), the use of condom type male prophylactic devices is increasing. It would be desirable if a way existed whereby a condom could be placed on the penis in an entirely sterile manner.

According to the present invention, there is provided a combination of a condom and a condom applicator wherein the condom is maintained within the applicatior with part of the condom wall held against an inner wall surface of the applicator by atmospheric pressure.

Also according to the invention, there is provided a method of manufacturing a condom applicator in combination with a condom including placing the closed end of a conventional condom within the applicator, stretching and rolling a portion of the open end of the condom over an open end of the applicator, substantially evacuating the air between the applicator wall and the condom wall, and sealing the orifice by which the air is evacuated.

In the use of such a combination, the applicator with condom therein is placed over the penis and then air is allowed to re-enter the space between the external wall of the closed end portion and part of the shaft portion of the condom and the internal wall of the applicator. The applicator can then be readily removed. Up to that time, the external surface of the closed end of the condom has been maintained entirely sterile.

The invention will be better understood from the following non-limiting description of an example thereof, given with reference to the accompanying drawings, in which: -

Figure 1 is a side elevation of one example of condom applicator in accordance with the present invention; .

Figure 2 is a plan view of the condom applicator shown in Figure 1;

Figure 3 is an end view of the condom applicator shown in Figure 1;

Figure 4 is a view similar to Figure 2 but illustrating the applicator in combination with a condom just prior to initial insertion of the condom within the applicator;

Figure 5 is a view similar to Figure 2 but illustrates the condom located within the applicator just prior to the application of negative pressure.

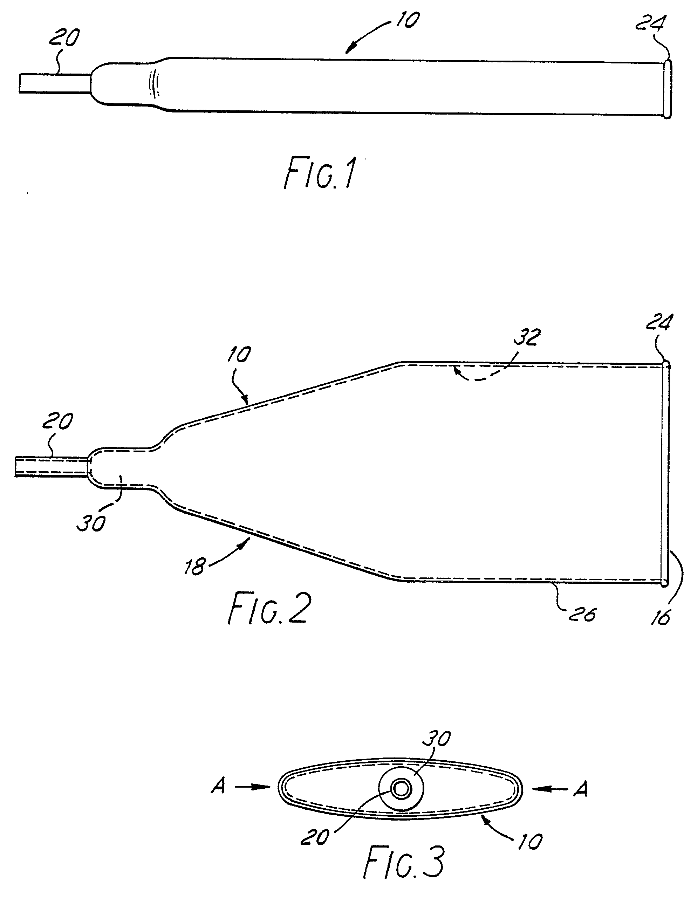

The condom applicator 10 illustrated in Figures 1 - 3 is made of a flexible, moldable, deformable, plastics material such as polypropylene and comprises a tapered container which in its undeformed state is substantially oval in cross-section and which has an open end 16 and an end which tapers as indicated at 18 in Figure 2. The opposite end has a tube 20 connected thereto. The open end has a rim 24. The purpose of the tube 20 is to permit the attachment thereto of a conduit (not shown) by which a negative pressure, relative to atmospheric, may be applied to the interior of the condom applicator for the purpose which will be later described.

As seen in Figure 2, the conndom applicator has a tapering shape as seen in plan with parallel sides 26 and tapering sides 18 leading to a recess 30 at the end opposite and remote from the open end 16. The recess 30 is dimensioned to encompass the reservoir tip of the male condom.

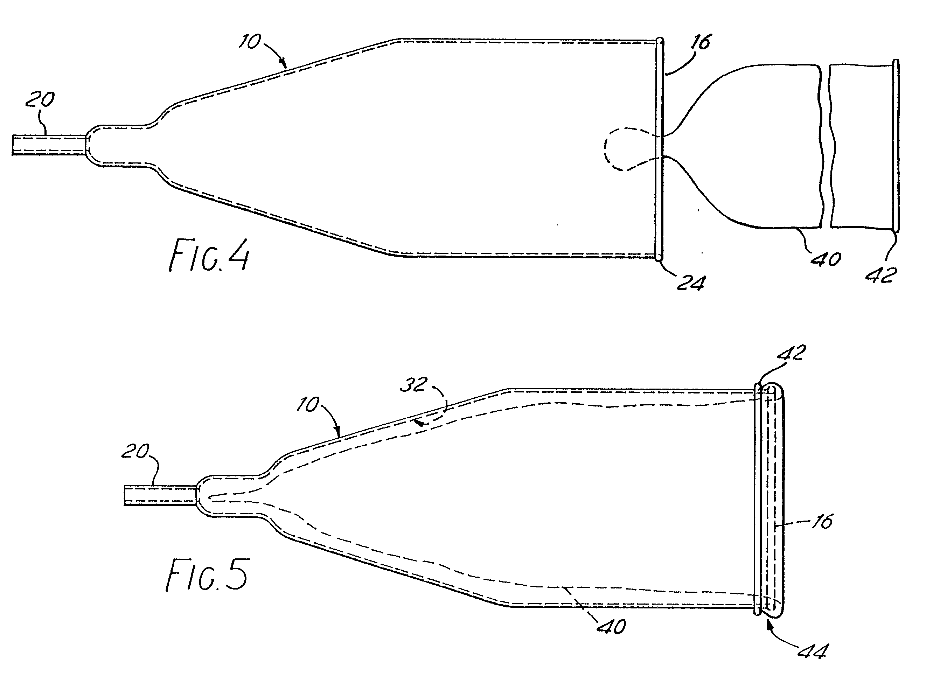

As has been mentioned, the material of the condom applicator is deformable. Hence, by applying pressure as shown by the arrows A in Figure 3, the applicator can be deformed to a substantially circular cross-sectional shape. This permits it to be readily placed over an erect penis. However, the normal undeformed cross-section of a flattened oval means that the space needed to package a given number of condom applicators is reduced.

The manner of use of a condom with a condom applicator will be understood from a consideration of Figures 4 and 5.

Initailly, for example in the factory or in an assembly area, a condom 40 is assembled together with a corresponding applicator 10. The condom is placed within the applicator 10 and the rim 42 of the condom is rolled around the rim 24 of the applicator as indicated at 44 in Figure 5. For this to be achieved it is necessary that the condom be considerably stretched, and hence once the condom is within the applicator, the tight gripping of the condom rim 42 around the free end 16 of the applicator 10 renders this connection substantially airtight. Thereafter, a conduit is attached to the tube 20 and a negative pressure relative to atmospheric or relative to the ambient pressure in which the assembly is being conducted is applied. As a result of this, the condom is stretched and caused to adhere closely to the internal wall 32 of the applicator 10. Thereafter the tube 20 is closed by being crimped or clamped or a sealant is applied to close the tube. This prevents the condom moving away from the internal wall 32 of the applicator when the negative pressure is removed. The applicator/condom combination is then in condition for transport and distribution and is in the condition in which it may be sold.

It will be understood that the aforesaid relatively simple assembly operations can be conducted in a sterile environment and the external surface of the condom and the internal wall of the applicator are entirely sterile at that time.

In use, once the applicator has been placed on the erect penis, the tube 20 is broken off or opened, and the open end portion of the condom is unrolled from around the rim 24 of the applicator and caused to engage the penis shaft. The breaking off of the tube destroys the partial vacuum between condom and applicator and the elasticity of the condom causes it to immediately grip the penis. The applicator is then removed and discarded.

It will be seen that the foregoing particular description and illustrations show a substantially sterile condom/applicator combination which can be readily assembled and transported, and whose manner of use is simple and effective.

## Claims

1. In combination an applicator and a male prophylactic condom wherein said applicator comprises a container having an open end which tapers to a recess at the opposite end, a rim circumscribing said open end, and a tube extending from said recess, a condom having an open and closed end located with said applicator, said condom open ended portion stretched and rolled over said applicator rim wherein air is evacuated through said tube to cause said condom to adhere closely to the internal wall of said applicator.

2. The combination of Claim 1 wherein said applicator is made of a flexible, moldable, deformable plastics material.

3. The combination of Claim 2 wherein said applicator and condom combination prior to use is undeformed and substantially oval in cross-section.

4. The method of applying a prophylactic condom in place on the male penis in a sterile manner comprising:

a) locating a condom having an open and closed end within an applicator, said applicator comprising a container having an open end which tapers to a recess at the opposite end, a rim circumscribing said open end, and a tube extending from said recess, and said condom open ended portion stretched over said applicator rim;

b) attaching a conduit to said tube extending from said applicator and causing negative pressure to be applied whereby said condom is stretched and adheres closely to the internal wall of said applicator;

c) sealing said tube;

d) placing said applicator and condom combination on an erect penis; and

e) unsealing said tube and unrolling the open ended portion of said condom from said applicator rim causing the condom to grip the penis.

5. The method of Claim 4 wherein said applicator is made of a flexible moldable, deformable plastics material.

6. The method of Claim 5 wherein said applicator and condom combination prior to use is undeformed and substantially oval in cross-section.

FIG.1

FIG.2

FIG.3

FIG.4

FIG.5

European Patent
Office

Application number

| | DOCUMENTS CONSIDERED TO BE RELEVANT | | EP 88306080.8 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | US - A - 4 281 648 (M.MAURICE ROGERS)<br><br>    * Column 2, line 33 - column 4, line 54; fig. 1 *<br><br>    ---- | 1 | A 61 F 5/42 |
| | | | **TECHNICAL FIELDS SEARCHED (Int. Cl.4)** |
| | | | A 61 F 5/00 |

The present search report has been drawn up for all claims

| Place of search | Date of completion of the search | Examiner |
|---|---|---|
| VIENNA | 27-09-1988 | GERSTBACH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82